# EUROPEAN PATENT SPECIFICATION

(11) **EP 3 763 339 B1**
(45) Date of publication and mention of the grant of the patent: **10.05.2023**
(21) Application number: 18934893.1
(22) Date of filing: 26.09.2018
(51) Int. Cl.: A61F 2/07, A61F 2/90

(54) **STENT AND STENT GRAFT**
STENT UND STENTPROTHESE
ENDOPROTHÈSE ET GREFFE D'ENDOPROTHÈSE

(43) Date of publication of application: 13.01.2021
(73) Proprietor: Japan Lifeline Co., Ltd., Tokyo 140-0002 (JP)
(72) Inventor: SAKAI, Masamune, Tokyo 140-0002 (JP)
(74) Representative: D Young & Co LLP
(86) International application number: PCT/JP2018/035548
(87) International publication number: WO 2020/065743

(56) References cited:
- JP-A- 2005 110 778
- JP-A- 2005 110 778
- JP-A- 2018 505 720
- US-A- 5 897 589

## Description

### Technical Field

The invention relates to a stent to be applied, for example, to a tubular organ in a body, such as a digestive tract or a blood vessel, and relates to a stent graft that includes the stent.

### Background Art

A stent to be applied to (placed in) a tubular organ in a body (such as a digestive tract or a blood vessel) and a stent graft that includes the stent are used for the following example applications. For example, the stent and the stent graft are used for preventing dilatation, rupture, or narrowing of the tubular organ attributed to deterioration in the tubular organ. In addition, for example, the stent and the stent graft are used to push open a lumen of the tubular organ in the body, which is displaced and thus narrowed by a tumor of the tubular organ and an organ positioned close to the tubular organ. The stent to be applied to a tubular organ in the body generally has a mesh structure including one or more wire members (e.g., see PTL 1).

### Citation List

### Patent Literature

PTL 1: Japanese Unexamined Patent Application Publication (Published Japanese Translation of PCT Application) No. 2009-501049

### Summary of Invention

Incidentally, it is generally demanded that, upon placing such a stent inside a tubular organ in a body as described above, the stent be able to reduce a risk of damaging an inner wall of the tubular organ. It is desirable to provide a stent that makes it possible to reduce a risk of damaging an inner wall of a tubular organ in a body and a stent graft that includes the stent.

**Prior art includes:** JP 2005 110778 A and US 5 897589 A. Each of these prior art disclosures describe a prior art device.

Embodiments of the present disclosure are defined by the appended claims.

### Brief Description of Drawings

[FIG. 1] FIG. 1 is a schematic perspective view of an exemplary outline configuration of a stent according to one embodiment of the invention.
[FIG. 2] FIG. 2 is a schematic development view of an exemplary detailed configuration of a main part of the stent illustrated in FIG. 1.
[FIG. 3] FIG. 3 is a schematic diagram illustrating an exemplary detailed configuration of a region near a wire member fixation part illustrated in FIG. 2.
[FIG. 4] FIG. 4 is a schematic diagram illustrating an example of a method of forming the wire member fixation part illustrated in FIG. 3.
[FIG. 5] FIG. 5 is a schematic diagram illustrating an exemplary configuration of a region near a fixing part of a wire member of a stent according to a comparative example not being covered by the invention.
[FIG. 6] FIG. 6 is a schematic perspective view of an exemplary outline configuration of a stent graft according to an application example.
[FIG. 7] FIG. 7 is a schematic diagram illustrating an exemplary configuration of a region near the wire member fixation part of a stent according to other modification examples not being covered by the invention.

### Description of Embodiments

Hereinafter, embodiments of the invention are described in detail with reference to the drawings. It is to be noted that the description is given in the following order.
1. Embodiment (an example of a stent in which a wire member fixation part that fixedly joins an end of a wire member includes a fiber wire member)
2. Application Example (an example of a case where the stent according to the embodiment is applied to a stent graft)
3. Other Modification Examples

### <1. Embodiment>

### [A. Outline Configuration]

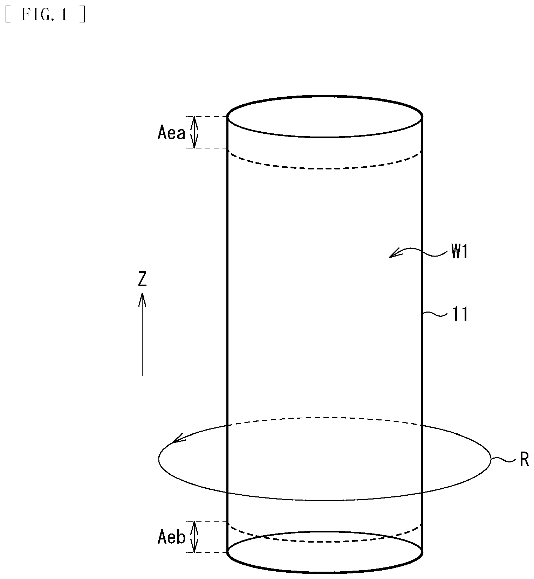

FIG. 1 is a schematic perspective view of an exemplary outline configuration of a stent (a stent 11) according to one embodiment of the invention. The stent 11 is an instrument to be applied to a tubular organ in a body (e.g., a digestive tract such as the large intestine or a blood vessel such as the aorta), and is used to, for example, push open a lumen of the tubular organ in the body which is narrowed by a tumor, as will be described later. Specifically, the stent 11 is to be placed in a site to be treated (in the tubular organ in the body as described above). It is to be noted that the stent 11 corresponds to a specific example of a "stent" in the invention and encompasses a covered stent.

The stent 11 has a tubular (cylindrical) structure that extends along its axial direction Z (Z axis direction), as illustrated in FIG. 1. The stent 11 also has both end regions (end regions Aea and Aeb) and a non-end region (an intermediate region positioned between the end regions Aea and Aeb) along its axial direction Z (see FIG. 1). The stent 11 has a length along the axial direction Z of, for example, about 3 cm to about 25 cm. On the other hand, the stent 11 has, when expanded, an outer diameter of, for example, about 10 mm to about 50 mm.

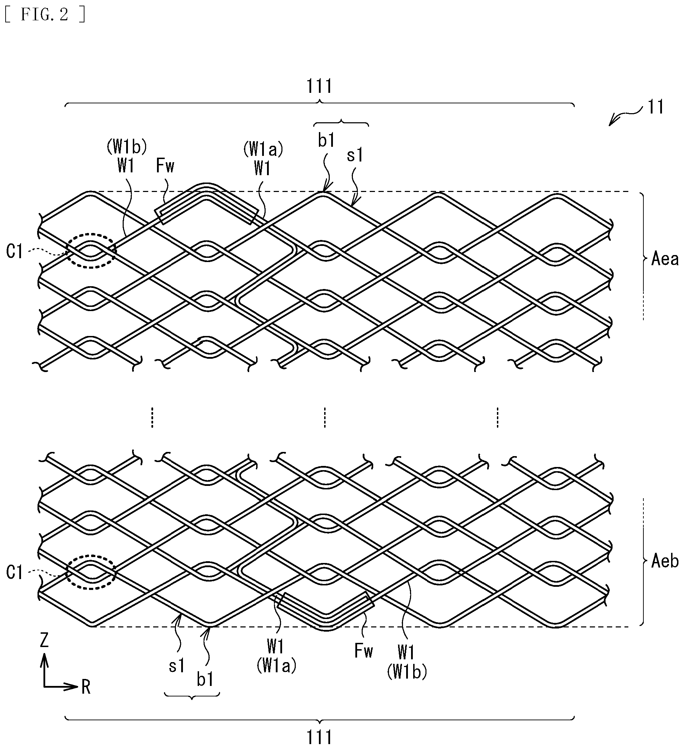

In an example illustrated in FIG. 1, the stent 11 includes a single type of wire members W1 (wires), and has a tubular structure as described above. Specifically, in the present embodiment, the tubular structure includes a mesh structure, and the tubular mesh structure is formed by weaving the wire members W1 in a predetermined pattern (a mesh pattern to be described later). It is to be noted that the mesh structure (a weaving pattern of the wire members W1), etc. of the stent 11 will be described in detail later (FIG. 2).

Here, a metal wire member may be preferable as a material of the wire members W1. In particular, a shape-memory alloy may be preferably employed. The shape-memory alloy is provided with a shape-memory effect, superelasticity, etc. by a thermal treatment. However, stainless-steel, tantalum (Ta), titanium (Ti), platinum (Pt), gold (Au), tungsten (W), etc. may be used as the material of the wire members W1, depending on applications. As the above-described shape-memory alloy, for example, an alloy of nickel (Ni) and Ti, an alloy of copper (Cu), zinc (Zn), and X (where X is aluminum (Al), iron (Fe), etc.), an alloy of Ni, Ti, and X (where X is Fe, Cu, vanadium (V), cobalt (Co), etc.), etc. may be preferably used. It is to be noted that, for example, a synthetic resin, etc. may be used as such wire members W1. Moreover, a metal wire member having a surface covered with Au, Pt, etc. by a method such as plating may be used as the wire members W1. Alternatively, a composite wire member in which a core material is covered with an alloy may be used as the wire members W1. The core material includes a material that does not transmit radiation, such as Au or Pt.

### [B. Detailed Configuration]

Next, with reference to FIGs. 2 to 4, description is given on an exemplary detailed configuration of the stent 11 illustrated in FIG. 1 (a configuration example of the above-described mesh structure, etc.). FIG. 2 is a schematic development view of an exemplary detailed configuration of a main part (the vicinity of the end regions Aea and Aeb) of the stent 11, and illustrates the exemplary detailed configuration along each of the axial direction Z and a circumferential direction R illustrated in FIG. 1.

Firstly, the stent 11 includes a single type of mesh structure 111 as illustrated in FIG. 2. That is, the stent 11 includes the mesh structure 111 including the wire members W1 described above. The mesh structure 111 constitutes the above-described tubular structure and extends in the axial direction Z (over the entire length) of the stent 11. Note that the wire member W1 may have a wire diameter of, for example, about 0.30 mm.

The mesh structure 111 has a wavy shape in which the wire member W1 including a straight part s1 and a bent part b1 forms a top in its bent part b1 as illustrated in FIG. 2. In addition, the mesh structure 111 is formed by the wire members W1 intersecting at their respective straight parts s 1. Accordingly, the mesh structure 111 has intersections (wire member intersections) where the straight parts s1 of the wire members W1 intersect each other.

Moreover, the mesh structure 111 has coupling parts C1 (hooking parts) in which the wire members W1 are coupled to (engaged with) each other at their respective bent parts b1. That is, the mesh structure 111 is formed by the mesh patterns that are coupled to each other along the axial direction Z. The mesh pattern is formed by the wire member W1, forming the above-described wavy shape, that progresses along the circumferential direction R.

Described specifically now is the respective mesh patterns which are as follows. That is, the mesh patterns are each formed by the wire member W1 making two laps of loops along the circumferential direction R while forming the wavy shape. More specifically, the mesh pattern of each row includes a first loop as the loop of the first lap and a second loop as the loop of the second lap. In the mesh pattern of each row, the first loop and the second loop have the wavy shapes whose phases are shifted by half a pitch (1/2 pitches) from each other along the circumferential direction R. Thus, in the mesh pattern of each row, the above-described intersections are positioned side by side along the circumferential direction R. It is to be noted that, in such intersections, the first loop and the second loop so intersect as to go up and down alternately.

### (Wire Member Fixation Part Fw)

Here, the stent 11 according to the present embodiment includes a wire member fixation part Fw in the mesh structure 111 as illustrated in FIG. 2. The wire member fixation part Fw fixedly joins an end of the wire member W1.

Referring to FIG. 2, the end regions Aea and Aeb of the stent 11 include the wire member fixation parts Fw as described below. That is, once the second loop is formed in the mesh pattern of the first row, the wire member W1 so progresses along the circumferential direction R as to be parallel with the first loop while forming the wavy shape that has the same phase as the first loop, until the wire member W1 makes a transition to the mesh pattern of the second row. Accordingly, the wire member W1 progresses while being positioned adjacent to a wire member that forms the first loop, until the wire member W1 makes the transition to the mesh pattern of the second row from the formation of the second loop. The end of the wire member W1 is fixedly joined at the wire member fixation part Fw to the wire member that is adjacent thereto in the above-described fashion. In other words, the wire member fixation part Fw fixedly joins the end of the wire member W1 (a wire member W1a in an example illustrated in FIG. 2) and the wire member W1 that is the same as the wire member W1a (a wire member W1b in the example illustrated in FIG. 2) to each other.

Note that the stent 11 according to the present embodiment includes the single type of wire members W1 as described above at least in the end regions Aea and Aeb. Hence, the wire member W1a and the wire member W1b are the same wire member. However, for example, the stent may include a plurality of types of wire members. In this case, the wire member W1a and the wire member W1b described above may be different wire members. That is, in that case, the wire member fixation part Fw fixedly joins, to each other, the end of the wire member W1a and another wire member W1b that is different from the wire member W1a.

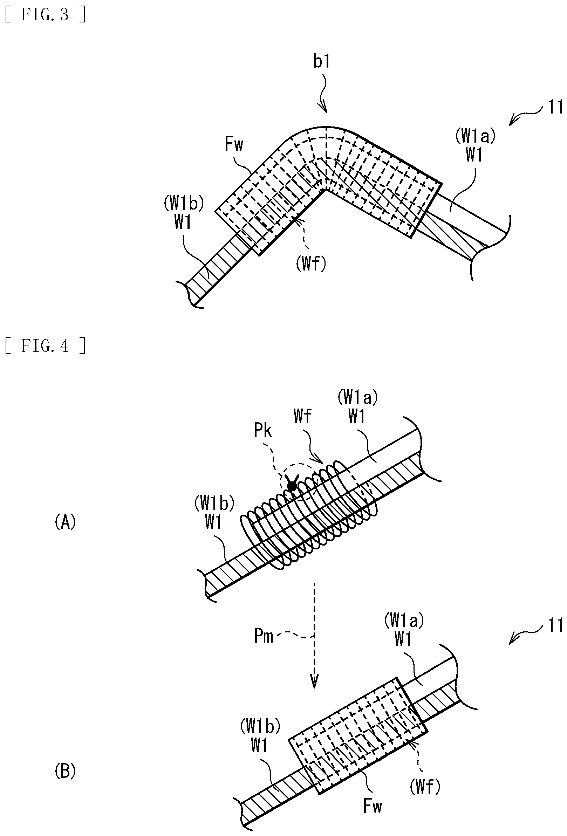

FIG. 3 schematically illustrates an exemplary detailed configuration of a region near the wire member fixation part Fw. Specifically, the end of the wire member W1a described above and a non-end part of the wire member W1b described above are fixedly joined to each other at the wire member fixation part Fw in an example illustrated in FIG. 3.

As illustrated in FIG. 3, the wire member fixation part Fw includes a fiber wire member Wf. The fiber wire member Wf includes the end of the wire member W1a and the wire member W1b (the non-end part of the wire member W1b) that are wound around each other. Specifically, the fiber wire member Wf has a configuration in which the wire members W1a and W1b are wound a plurality of times along a circumferential direction of the wire members W1a and W1b. In particular, in the present embodiment, at least a portion of the fiber wire member Wf in the wire member fixation part Fw includes a molten layer, which will be described later in detail.

The wire member fixation part Fw is disposed in a region, of the wavy shape described above, that includes the top (the bent part b1) as illustrated in FIG. 3. In other words, the wire member fixation part Fw fixedly joins the wire member W1 (W1a and W1b) in a region that includes the bent part b1 of the wire member W1. Specifically, according to the invention illustrated in FIG. 3, the wire member fixation part Fw is so disposed as to include a region of the straight part s1 in which the end of the wire member W1a has extended beyond the top (the bent part b1) of the wavy shape. According to the invention, the wire member fixation part Fw is disposed across: the region near the top of the wavy shape; a region of the straight part s1 positioned at a part that is before the top (a part positioned on the right side of the bent part b1 in FIG. 3); and a region of the straight part s1 positioned at a part that is beyond the top (a part positioned on the left side of the bent part b1 in FIG. 3).

Note that the fiber wire member Wf can include, for example, the following material (fiber material). Namely, it is possible to use a resin or the like having durability and low tissue reaction for the fiber material that constitutes the fiber wire member Wf, examples of which may include: polyolefin such as polyethylene terephthalate (PET), polyethylene, polypropylene, or ethylen-α-olefin copolymer; polyester such as polyamide, polyurethane, polybutylene terephthalate, polycyclohexane terephthalate, or polyethylene-2, 6-naphthalate; a vinyl resin such as polyvinyl chloride, vinyl acetate, or ethylene-vinyl acetate copolymer; and a fluororesin such as polyethylene fluoride or polypropylene fluoride. Examples of the resin may also include polyamide, polyamide elastomer, polyurethane, a silicone resin, and a natural rubber. In particular, among the above-described materials, the polyester such as polyethylene terephthalate, the fluororesin such as polyethylene fluoride or polypropylene fluoride, or the silicone resin can be preferably used, which are chemically stable, have great durability, and cause a low tissue reaction.

Here, for example, it is possible to form the wire member fixation part Fw as illustrated in (A) and (B) of FIG. 4. (A) and (B) of FIG. 4 schematically illustrate an example of a method of forming the wire member fixation part Fw.

The method of forming the wire member fixation part Fw first involves winding the fiber wire member Wf a plurality of times along the circumferential direction of the end of the wire member W1a and the wire member W1b (the end or the non-end part of the wire member W1b), with the end of the wire member W1a and the wire member W1b (the end or the non-end part of the wire member W1b) being bundled, as illustrated by way of example in (A) of FIG. 4. At this time, ends of the fiber wire member Wf wound a plurality of times are joined to each other (see a knot Pk illustrated in (A) of FIG. 4).

Next, the thus-wound fiber wire member Wf is melted, as denoted by a broken line arrow Pm between (A) and (B) of FIG. 4, for example. This forms the molten layer of the fiber wire member Wf as illustrated by way of example in (B) of FIG. 4. At this time, it is preferable that, as a result of the melting of the knot Pk as a whole and an outer surface of the fiber wire member Wf, the molten layer be formed only on the outer surface of the fiber wire member Wf and the inner side of the fiber wire member Wf be unmelted. In other words, it is preferable that only a portion of the fiber wire member Wf of the wire member fixation part Fw serve as the molten layer. The wire member fixation part of the stent 11 is thus formed as described above.

### [Workings and Effects]

### (A. Basic Operation)

The stent 11 is placed at a site to be treated (e.g., inside the tubular organ described above) upon treatment of a tumor or the like near a tubular organ in a body of a patient (e.g., a digestive tract such as the large intestine or a blood vessel such as the aorta). This achieves the following; namely, it is possible to prevent dilatation, rupture, or narrowing of the tubular organ attributed to deterioration in the tubular organ. In addition, for example, it is possible to push open a lumen of the tubular organ in the body, which is displaced and thus narrowed by a tumor of the tubular organ and an organ positioned close to the tubular organ.

At this occasion, specifically, the stent 11 is first placed in a predetermined delivery sheath in a state of being reduced in diameter, and the delivery sheath is inserted into the tubular organ to allow the stent 11 to be carried to the vicinity of an affected site. The stent 11 is then expanded by being deployed from the inside of the delivery sheath. Thus, the stent 11 is placed in the affected site (the site to be treated).

### (B. Workings and Effects of Stent 11)

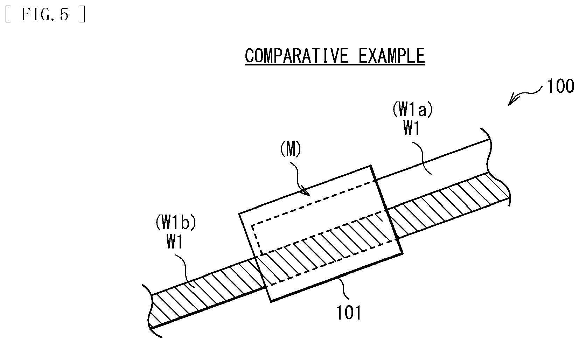

Next, with reference to FIG. 5 in addition to FIGs. 1 to 4, workings and effects of the stent 11 are described in detail in comparison with a comparative example.

### (B-1. Comparative Example)

FIG. 5 schematically illustrates an exemplary configuration of a region near a fixing part (a fixing part 101) of the wire member W1 (W1a and W1b) of a stent (a stent 100) according to a comparative example not being covered by the invention.

In the stent 100 according to the comparative example, an end of the wire member W1a and the wire member W1b (the non-end part of the wire member W1b) are fixed to each other at the fixing part 101 that uses mechanical coupling of a metal component (so-called "crimping") as illustrated in FIG. 5 (see reference numeral M in FIG. 5). Specifically, in the fixing part 101 according to the comparative example, the wire member W1a and the wire member W1b are fixed by the "crimping" that uses, for example, a SUS (stainless steel) pipe.

The stent 100 according to the comparative example thus fixes the wire member W1a and the wire member W1b in the fixing part 101 that uses the mechanical coupling of the metal component, causing formation of an edge or a protrusion of the metal component at the fixing part 101. Hence, the stent 100 according to the comparative example can increase a risk of damaging the inner wall of the tubular organ in the body (such as the blood vessel wall).

### (B-2. Present Embodiment)

In contrast, in the stent 11 according to the present embodiment, the wire member fixation part Fw that fixedly joins the end of the wire member W1 (W1a) and the wire member W1 (W1b) to each other includes the fiber wire member Wf in which the wire members W1a and W1b are wound around each other as illustrated in FIGs. 2 to 4. Thus, the present embodiment makes it difficult to form (desirably, eliminates the formation of) the edge or the protrusion at the wire member fixation part Fw as compared with a case, such as the comparative example described above, in which the wire members W1a and W1b are fixed to each other by using the mechanical coupling of the metal component, for example. Hence, the present embodiment makes it possible to reduce the risk of damaging the inner wall of the tubular organ in the body described above, as compared with, for example, the comparative example described above.

In addition, the wire member fixation part Fw in the present embodiment includes the fiber wire member Fw which is flexible, allowing the wire member fixation part Fw to have a flexible structure. Hence, it is also possible to make deformation of the stent 11 (and the stent graft 1 described later) less likely to be hindered.

In addition, for example, unlike the fixing part 101 according to the comparative example described above (unlike a fixing method that uses the so-called "crimping"), the wire member fixation part Fw of the present embodiment eliminates a sharp change in hardness. Hence, the present embodiment makes it difficult to generate (desirably, eliminates the generation of) bending resulting from concentration of a stress caused by the sharp change in the hardness. Further, for example, unlike the fixing part 101 according to the comparative example described above, the wire member fixation part Fw of the present embodiment uses no metal material. Hence, it is possible to prevent generation of corrosion resulting from a contact of dissimilar metals.

In addition, the wire member fixation part Fw of the present embodiment is disposed in the region, of the wavy shape described above, that includes the top (the bent part b1). Thus, a positional displacement of the wire member fixation part Fw is suppressed (is desirably prevented), making it difficult for the end of the wire member W1a described above to be pulled out from the wire member fixation part Fw. Hence, it is possible to further reduce the risk of damaging the inner wall of the tubular organ in the body.

Note that the following issue occurs if the fixing part 101 is disposed in the region that includes the bent part b1 as with, for example, the present embodiment, in a case where the end of the wire member W1a and the non-end part of the wire member W1b are fixed (at the fixing part 101) by the "crimping" that uses the SUS pipe as with the comparative example described above. Namely, in the comparative example, disposing the fixing part 101 in the region that includes the bent part b1 makes it difficult for the bent part b1 to be subjected to bending (or makes it not possible for the bent part b1 to be subjected to bending), due to high hardness of the fixing part 101. Hence, it is not possible to reduce the diameter of the stent 100 sufficiently and to place the stent 100 inside a delivery catheter. Accordingly, it is not possible for the comparative example to dispose the fixing part 101 in the region that includes the bent part b1, due to the "crimping" that uses the SUS pipe. In contrast, according to the present embodiment, the wire member fixation part Fw uses the fiber wire member Wf as described above, preventing hardness of the wire member fixation part Fw from becoming high and thus making it easier to bend the bent part b1 even in a case where the wire member fixation part Fw is disposed in the region that includes the bent part b1. Thus, it is possible to reduce the diameter of the stent 11 sufficiently and to place the stent 11 inside the delivery catheter. Accordingly, unlike the comparative example described above, the present embodiment uses the fiber wire member Wf to allow the wire member fixation part Fw to be disposed in the region that includes the bent part b1.

In addition, in the present embodiment, the wire member fixation part Fw is so disposed as to include the region of the straight part s1 in which the end of the wire member W1a has extended beyond the top of the wavy shape described above. This achieves the following; namely, the wire members W1a and W1b are disposed to be adjacent to each other (see FIG. 3) at the top (the bent part b1), further suppressing (desirably, preventing) the positional displacement of the wire member fixation part Fw. This makes it further difficult for the end of the wire member W1a to be pulled out from the wire member fixation part Fw. Hence, it is possible to reduce the risk of damaging the inner wall of the tubular organ in the body even further consequently.

Further, at least a portion of the fiber wire member Wf of the wire member fixation part Fw includes the molten layer. This achieves the following; namely, for example, the outer surface of the fiber wire member Wf and the knot Pk as a whole in the wire member fixation part Fw are melted, making it difficult (desirably, making it not possible) for the fiber wire member Wf of the wire member fixation part Fw to come loose. Thus, the formation of the edge or the protrusion described above becomes further difficult to occur at the wire member fixation part Fw. Hence, it is possible to further reduce the risk of damaging the inner wall of the tubular organ in the body.

### <3. Application Example>

Next, description is given on an example of applying the stent (the stent 11) according to the foregoing embodiment to a stent graft.

### [A. Configuration]

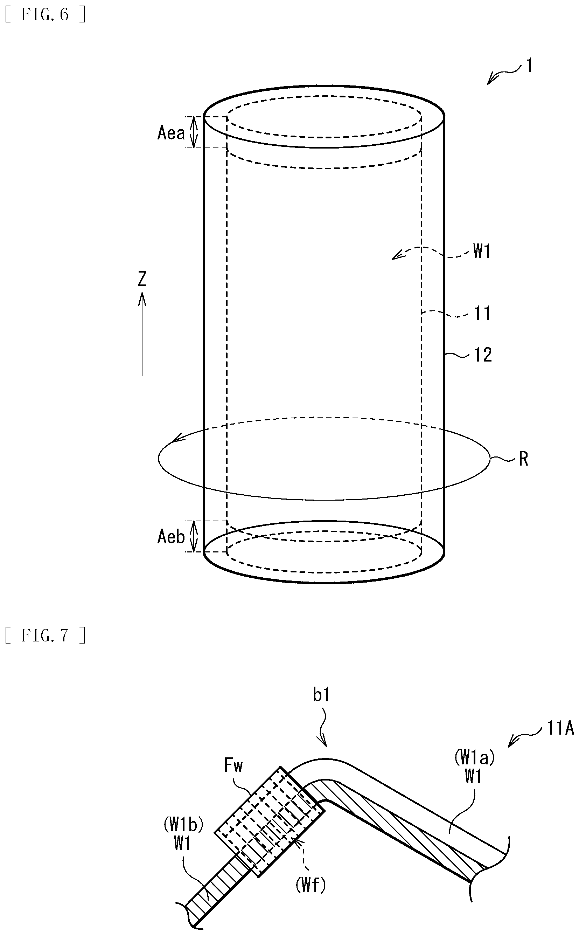

FIG. 6 is a schematic perspective view of an exemplary outline configuration of a stent graft (a stent graft 1) according to the present application example. The stent graft 1 includes the stent 11 according to the invention and a tubular member 12 to be described below. The stent graft 1 is a medical device to be applied, for example, to a blood vessel such as the aorta described above.

### (Tubular Member 12)

The tubular member 12 has a tubular (cylindrical) shape as illustrated in FIG. 6. The tubular member 12 is so disposed as to cover (coat) at least a portion of the stent 11. Specifically, the tubular member 12 is so disposed as to cover an outer circumference side of the stent 11 in this example.

Moreover, the tubular member 12 is coupled to the stent 11 by a method such as sewing, attaching, or welding, so as to coat the stent 11. It is to be noted that a coupling part at which the tubular member 12 and the stent 11 are coupled to each other is provided as appropriate at a location such as the end regions Aea and Aeb or the non-end region (the intermediate region) of the stent 11.

Here, in an example illustrated in FIG. 6, the stent 11 is disposed in the entire region (the end regions Aea and Aeb and the intermediate region) along the axial direction Z of the tubular member 12. However, this is non-limiting, and the stent 11 may be disposed only in a partial region along the axial direction Z of the tubular member 12. In other words, the stent graft 1 may have, along its axial direction Z, a region (a stent disposed region) in which the stent 11 is disposed and a region (a stent non-disposed region) in which the stent 11 is not disposed.

Examples of the tubular member 12 include: a resin that is formed into a tubular shape by a molding method such as extrusion molding or blow molding; a tubular-shaped knitted or woven material including a fiber of resin or a super-fine metal wire; a tubular-shaped non-woven fabric including a resin or super-fine metal; a tubular-shaped resin sheet or a tubular-shaped porous sheet; a structure derived from a resin that has been dissolved in a solvent and is formed into a thin tubular shape; etc.

Here, as the above-described knitted or woven material, a known material knitted or woven in plane weave, twill weave, etc. may be used. Alternatively, a knitted or woven material with a crimp such as that subjected to a crimping process may also be used.

Moreover, as the above-described resin, for example, a resin having durability and causing a low tissue reaction may be used. Examples of the resin may include: polyolefin such as polyethylene, polypropylene, or an ethylene-α-olefin copolymer; polyester such as polyamide, polyurethane, polyethylene terephthalate, polybutylene terephthalate, polycyclohexane terephthalate, or polyethylene-2, 6-naphthalate; a vinyl resin such as polyvinyl chloride, vinyl acetate, or an ethylene-vinyl acetate copolymer; and a fluororesin such as polyethylene fluoride or polypropylene fluoride. Examples of the resin may also include polyamide, polyamide elastomer, polyurethane, a silicone resin, and a natural rubber. In particular, among the above-described materials, the polyester such as polyethylene terephthalate, the fluororesin such as polyethylene fluoride or polypropylene fluoride, or the silicone resin can be preferably used, which are chemically stable, have great durability, and cause a low tissue reaction.

Note that, for example, the material constituting the tubular member 12 and the material (the fiber material) constituting the fiber wire member Wf of the wire member fixation part Fw described in the embodiment may be the same as each other in the stent graft 1 according to the present application example. In other words, the fiber wire member Wf of the wire member fixation part Fw may include a fiber material that is the same in kind as the material constituting the tubular member 12, such as that described above.

### [B. Workings and Effects]

Basically, it is also possible for the stent graft 1 according to the present application example to obtain effects similar to those of the embodiment by workings similar to those of the embodiment.

In addition, in particular, the fiber wire member Wf of the wire member fixation part Fw may include the fiber material that is the same in kind as that of the tubular member 12 as described above. In this case, the following is achieved; namely, using the materials (the fiber materials) which are the same in kind as each other for the fiber wire member Wf and the tubular member 12 facilitates the manufacture of the stent graft 1 and allows for a reduction of manufacturing costs.

### <3. Other Modification Examples>

The invention has been described above with reference to the embodiment and the application example; however, the invention is not limited to the embodiment, etc. described above, and is modifiable in a variety of ways.

For example, shapes, locations, sizes, numbers, materials, etc. of the respective members described in the foregoing embodiment, etc. are non-limiting, and may respectively be any other shape, location, size, number, material, etc. Specifically, for example, the tubular member may cover the inner circumference side of the stent or may cover both the inner circumference side and the outer circumference side of the stent.

In addition, the foregoing embodiment, etc. have been described with reference to an example case where at least a portion of the fiber wire member of the wire member fixation part includes the molten layer, but this case is non-limiting. That is, for example, the fiber wire member of the wire member fixation part may not be the molten layer. According to a configuration not covered by the invention, the wire member fixation part may be disposed in a region other than the region of the straight part in which the end of the wire member has extended beyond the top of the wavy shape described above. Additionally, the fiber wire member of the wire member fixation part may include a fiber material that is different in kind from that of the tubular member of the stent graft.

Note that a position at which the wire member fixation part is disposed may be as illustrated by way of example in FIG. 7. FIG. 7 schematically illustrates an exemplary configuration of a region near the wire member fixation part Fw of a stent (a stent 11A) according to other modification examples not being covered by the invention. In an example illustrated in FIG. 7, the wire member fixation part Fw is disposed in a region of the straight part s1 in which the end of the wire member W1a has extended beyond the top (the bent part b 1) of the wavy shape described above. Specifically, the wire member fixation part Fw is disposed only in the region of the straight part s1 in which the end of the wire member W1a has extended beyond the top (a part positioned on the left side of the bent part b 1 in FIG. 7), instead of the region near the top of the wavy shape and instead of the region of the straight part s1 positioned at a part that is before the top (a part positioned on the right side of the bent part b 1 in FIG. 7).

According to configurations not covered by the present invention, the arranged shape (the weaving pattern) of the wire members of the stent is not limited to those referred to in the foregoing embodiment, and may be any other arranged shape.

Further, although the foregoing application example has been described with reference to an example case where only one stent is disposed in the stent graft, this is non-limiting. Two or more stents may be individually disposed (for example, the two or more stents may be disposed along the axial direction Z while being separated from each other) in the stent graft.

Moreover, the foregoing embodiment, etc. have been described with reference to a case where the stent basically includes the single type of wire members (wires), but this case is non-limiting. That is, for example, the stent may include a plurality of types (two or more types) of wire members (wires) having wire diameters different from each other.

In addition, although the foregoing embodiment, etc. have been mainly described with reference to examples of the stent and the stent graft that are to be applied mainly to the treatment of the digestive tract such as the large intestine, the blood vessel such as the aorta, etc., this is non-limiting. That is, the stent and the stent graft according to the invention are each also applicable to treatment of, for example, any other digestive tract other than the large intestine or any other blood vessel other than the aorta (applicable to treatment of any other tubular organ in the body).

## Claims

1. A stent (11) to be applied to a tubular organ in a body, the stent comprising
a mesh structure (111) including one or more wire members (W1), and having a tubular structure that extends in an axial direction (Z) of the stent,
wherein
the mesh structure includes a wire member fixation part (Fw) that fixedly joins an end of the wire member (W1a) and the same wire member (W1b) as the wire member to each other, or fixedly joins the end of the wire member (W1a) and another wire member (W1b) to each other,
the wire member fixation part includes a fiber wire member (Wf), which is wound a plurality of times along the circumferential direction of the end of the wire member (W1a) and
the same wire member (W1b), with the end of the wire member (W1a) and the same wire member (W1b) being bundled, or a plurality of times along the circumferential direction of the end of the wire member (W1a) and the other wire member (W1b), with the end of the wire member (W1a) and the other wire member (W1b) being bundled;
and wherein ends of the fiber wire member (Wf) are joined to each other;
and wherein
the mesh structure has a wavy shape in which the wire member including a straight part (s1) and a bent part (b1) forms a top in the bent part, and
the wire member fixation part (Fw) is disposed across the region near the top of the wavy shape, a region of the straight part (s1) positioned at a part that is before the top, and a region of the straight part (s1) positioned at a part that is beyond the top.

2. The stent according to claim 1, wherein at least a portion of the fiber wire member in the wire member fixation part comprises a molten layer.

3. A stent graft (1) comprising:
a tubular member (12); and
at least one stent (11) according to claim 1, disposed in at least a portion of the tubular member.

4. The stent graft according to claim 3, wherein the fiber wire member includes a fiber material that is same in kind as that of the tubular member.

## Patentansprüche

1. Stent (11), der auf ein rohrförmiges Organ in einem Körper aufgebracht werden soll, der Stent umfassend
eine Geflechtstruktur (111), die ein oder mehrere Drahtelemente (W1) beinhaltet und eine rohrförmige Struktur aufweist, die sich in einer axialen Richtung (Z) des Stents erstreckt,
wobei
die Geflechtstruktur ein Drahtelementfixierungsteil (Fw) beinhaltet, das ein Ende des Drahtelements (W1a) und des gleichen Drahtelements (W1b) als das Drahtelement fixiert miteinander verbindet oder das Ende des Drahtelements (W1a) und ein anderen Drahtelement (W1b) fixiert miteinander verbindet,
das Drahtelementfixierungsteil ein Faserdrahtelement (Wf) beinhaltet, das eine Vielzahl von Malen entlang der Umfangsrichtung des Endes des Drahtelements (W1a) und des gleichen Drahtelements (W1b) gewickelt ist, wobei das Ende des Drahtelements (W1a) und das gleiche Drahtelement (W1b) gebündelt sind,
oder eine Vielzahl von Malen entlang der Umfangsrichtung des Endes des Drahtelements (W1a) und des anderen Drahtelements (W1b), wobei das Ende des Drahtelements (W1a) und das andere Drahtelement (W1b) gebündelt sind;
und wobei Enden des Faserdrahtelements (Wf) miteinander verbunden sind; und wobei
die Geflechtstruktur eine Wellenform aufweist, in der das Drahtelement, das einen geraden Teil (s1) und einen gebogenen Teil (b1) beinhaltet, eine Oberseite in dem gebogenen Teil ausbildet, und
das Drahtelementfixierungsteil (Fw) über der Region hinweg nahe der Oberseite der Wellenform angeordnet ist, eine Region des geraden Teils (s1), die an einem Teil positioniert ist, der vor der Oberseite liegt, und eine Region des geraden Teils (s1), die an einem Teil positioniert ist, der über die Oberseite hinaus liegt.

2. Stent nach Anspruch 1, wobei mindestens ein Abschnitt des Faserdrahtelements in dem Drahtelementfixierungsteil eine geschmolzene Schicht umfasst.

3. Stentimplantat (1), umfassend:
ein rohrförmiges Element (12); und
mindestens einen Stent (11) nach Anspruch 1, der in mindestens einem Abschnitt des rohrförmigen Elements angeordnet ist.

4. Stenttransplantat nach Anspruch 3, wobei das Faserdrahtelement ein Fasermaterial beinhaltet, das die gleiche Art wie die des rohrförmigen Elements ist.

## Revendications

1. Endoprothèse (11) destinée à être appliquée à un organe tubulaire dans un organisme, l'endoprothèse comprenant
une structure maillée (111) comportant un ou plusieurs éléments de fil (W1), et ayant une structure tubulaire qui s'étend dans une direction axiale (Z) de l'endoprothèse,
dans laquelle
la structure maillée comporte une partie fixation d'élément de fil (Fw) qui relie de manière fixe une extrémité de l'élément de fil (W1a) et le même élément de fil (W1b) que l'élément de fil l'un à l'autre, ou relie de manière fixe l'extrémité de l'élément de fil (W1a) et un autre élément de fil (W1b) l'un à l'autre,
la partie fixation d'élément de fil comporte un élément de fil de fibre (Wf), qui est enroulé une pluralité de fois le long de la direction circonférentielle de l'extrémité de l'élément de fil (W1a) et du même élément de fil (W1b), l'extrémité de l'élément de fil (W1a) et celle du même élément de fil (W1b) étant regroupées,
ou une pluralité de fois le long de la direction circonférentielle de l'extrémité de l'élément de fil (W1a) et de l'autre élément de fil (W1b), l'extrémité de l'élément de fil (W1a) et celle de l'autre élément de fil (W1b) étant regroupées ;
et dans laquelle les extrémités de l'élément de fil de fibre (Wf) sont jointes l'une à l'autre ; et dans laquelle
la structure maillée a une forme ondulée dans laquelle l'élément de fil comportant une partie droite (s1) et une partie coudée (b1) forme un sommet dans la partie coudée, et
la partie fixation d'élément de fil (Fw) est disposée dans toute la région proche du sommet de la forme ondulée, une région de la partie droite (s1) étant positionnée au niveau d'une partie qui est avant le sommet, et une région de la partie droite (s1) étant positionnée au niveau d'une partie qui est au-delà du sommet.

2. Endoprothèse selon la revendication 1, dans laquelle au moins une partie de l'élément de fil de fibre dans la partie fixation d'élément de fil comprend une couche fondue.

3. Endoprothèse couverte (1) comprenant :
un élément tubulaire (12) ; et
au moins une endoprothèse (11) selon la revendication 1, disposée dans au moins une partie de l'élément tubulaire.

4. Endoprothèse couverte selon la revendication 3, dans laquelle l'élément de fil de fibre comprend un matériau fibreux qui est du même type que celui de l'élément tubulaire.
